# EUROPEAN PATENT APPLICATION

(11) **EP 2 589 587 A1**
(43) Date of publication of application: **08.05.2013**
(21) Application number: 11382338.9
(22) Date of filing: 04.11.2011
(51) Int. Cl.: C07C 45/46, C07C 49/233, C07C 201/10, C07C 201/12, C07C 205/10, C07C 205/16, C07C 205/45, C07C 209/34

(54) **Synthesis of nitrogen substituted cyclopropanes**

(71) Applicant: Chemo Ibérica, S.A., 08028 Barcelona (ES)
(72) Inventor: Rasparini, Marcello, 27010 Cura Carpignano (IT); Taddei, Maurizio, 53035 Monteriggioni (IT); Cini, Elena, 50050 Gambassi Terme (IT); Minelli, Cosima, 21019 Somma Lombardo (IT); Turner, Nicholas, Manchester, Greater Manchester M20 3DH (GB); Hugentobler, Katharina Gloria, 64665 Alsbach-Hähnlein (DE)
(74) Representative: Ponti Sales, Adelaida

(57) **Abstract**

The invention is about a new synthetic pathway for the preparation of enantiomerically pure nitrogen substituted cyclopropanes, in particular the *trans-*cyclopropanamine (**1**), a key intermediate in the synthesis of Ticagrelor:

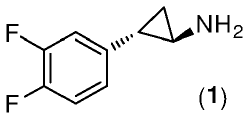

The invention also relates to the processes for preparing said compounds or their salts and the use of said compounds as intermediates in preparing pharmaceutically active ingredients.

## Description

### Field of the invention

The present invention relates to enantiomerically pure nitrogen substituted cyclopropanes, processes for preparing said compounds or their salts and the use of said compounds as intermediates in preparing pharmaceutically active ingredients.

### Background of the invention

Nitrogen substituted cyclopropanes are a particularly interesting class of compounds for the preparation of a large number of pharmaceutically active ingredients, such as 3-[7-[[(1*R*,2*S*)-2-(3,4-difluorophenyl)-cyclopropyl]amino]-5-(propylthio)-3*H*-1,2,3-triazolo[4,5-*d*] pyrimidin-3-yl]-5-(2-hydroxyethoxy)-(1*S*,2*S*,3*R,*5*S*)-cyclopentane-1,2-diol (also named Ticagrelor). This compound, produced by AstraZeneca (trade names Brilinta^{®} in the US, Brilique^{®} and Possia^{®} in the EU), is an allosteric antagonist of the ADP receptors of subtype P2Y₁₂, useful as platelet aggregation inhibitor and indicated for the prevention of thrombotic events.

Ticagrelor and other similar compounds are disclosed in WO 00/34283 A1 and WO 99/05143 A1, both assigned to AstraZeneca. These patents report a method for the preparation of the *trans*-cyclopropanamine (**1**), one of the key intermediates in Ticagrelor synthetic path:

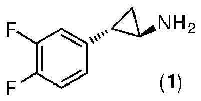

This method entails the preparation of the Oppolzer's camphor sultam derivative of 3,4-difluorophenyl acrylic acid followed by a cyclopropanation with diazomethane and a palladium catalyst.

On industrial scale, the use of this route of synthesis has several drawbacks caused by the high cost of the Oppolzer's camphor sultam and the known problems concerning the use of diazomethane, such as its explosiveness by shock, friction or heat and its high toxicity by inhalation.

A further synthetic method for the preparation of the *trans*-cyclopropanamine (**1**) is reported in WO 01/92200 A1, assigned to AstraZeneca, where (-)-menthyl-3,4-difluorophenyl acrylate is subjected to a diastereoselective Corey-Chaykovsky cyclopropanation. Problems related to this route are the high number of the synthetic steps, the use of some dangerous or expensive reagents such as NaN₃ or trimethylsulfoxonium iodide and an unacceptable low recovery (about 30%) of the desired intermediate.

Alternative methods for the preparation of the *trans-*cyclopropanamine (**1**) are reported in WO 2008/018822 A1 and WO 2008/018823 A1, both assigned to AstraZeneca. Despite being improvements over the prior art, the high number of steps reduces the overall efficiency of the industrial process.

Aims of this invention are to provide a new and short synthetic route for the industrial preparation of nitrogen substituted cyclopropananes, as well as to improve the yield and to overcome the known disadvantages of the prior art routes. A further aim of this work is to provide novel compounds that can be used in the process.

### Brief description of the invention

This and other purposes are achieved within the present invention, which regards enantiomerically pure nitrogen substituted cyclopropanes, their salts and the preparation thereof.

A first aspect of the present invention relates to a new synthetic route for preparing a nitrogen substituted cyclopropane.

In a first embodiment, the process of the invention comprises the following steps:
a) reducing a β-nitroketone (**D**) 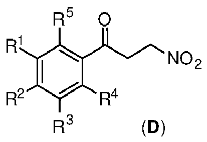 in which the substituents R¹, R², R³, R⁴, R⁵ are, independently from each other, hydrogen or a halogen, according to either:
   a1) non-enantioselective reduction conditions, to provide a β-nitroalcohol (**E**): 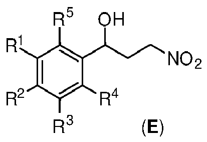 or:
   a2) enantioselective reduction conditions, to provide an enantiomerically enriched β-nitroalcohol (**Ea**): 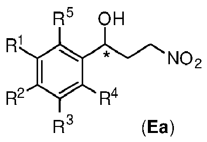
b) cyclizing the β-nitroalcohol (**E**) to yield a nitrocyclopropane (**G**) with a high trans/cis ratio, or cyclizing the enantiomerically enriched β-nitroalcohol (**Ea**) to yield an enantiomerically enriched nitrocyclopropane (**Ga)** with a high trans/cis ratio: 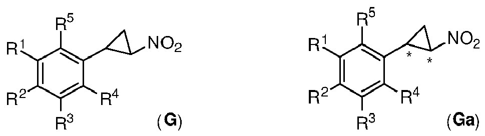
c) reducing the nitrocyclopropane (**G**) to the corresponding cyclopropylamine (**H**) with a high trans/cis ratio or a salt thereof, or reducing the enantiomerically enriched nitrocyclopropane (**Ga**) to the enantiomerically enriched cyclopropylamine (**Ha**) with a high trans/cis ratio, or a salt thereof:

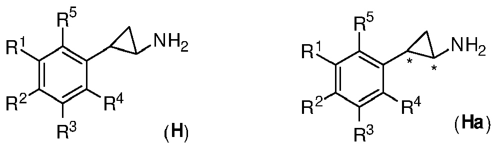

In case step a) is carried out according to non-enantioselective reduction conditions (reduction a1), an additional step d) may be performed, to convert the cyclopropylamine (**H**) into one of its pharmaceutically acceptable or unacceptable salts or in a salt with a chiral acid which may be recrystallized to enhance its diastereoisomeric and/or enantiomerc excess and provide the enantiomerically enriched cyclopropylamine (**Ha**) with a high trans/cis ratio or a salt thereof.

The cyclopropylamine (**Ha**), or one of its salts, may be further converted, in an additional step e), into Ticagrelor, or in an intermediate useful for its synthesis or one of its salts.

In a preferred embodiment, the invention relates to a process for preparing the cyclopropylamine (**I**) with a high trans/cis ratio, comprising:
a') reducing the β-nitroketone (**IV**): 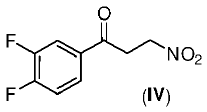 according to either:
a1') non-enantioselective reduction conditions, to provide the β-nitroalcohol (**III**): 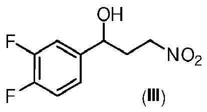 or:
a2') enantioselective reduction conditions, to provide an enantiomerically enriched β-nitroalcohol (**IIIa**): 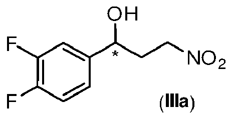
b') cyclizing the β-nitroalcohol (**III**) or the enantiomerically enriched β-nitroalcohol (**IIIa**) to yield a nitrocyclopropane (**II**) with a high trans/cis ratio, or the enantiomerically enriched nitrocyclopropane (**IIa**) with a high trans/cis ratio: 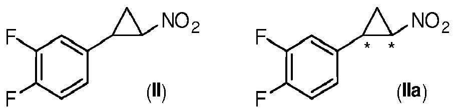
c') reducing the nitrocyclopropane (**II**) or the enantiomerically enriched nitrocyclopropane (**IIa**) to the corresponding cyclopropylamine (**I)** with a high trans/cis ratio, or to the enantiomerically enriched cyclopropylamine (**Ia**) with a high trans/cis ratio, or a salt thereof:

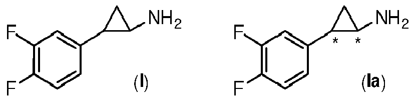

In case step a') is carried out according to non-enanctioselective reduction conditions (reduction a1') an additional step d') may be performed, to convert the cyclopropylamine (**I**) into one of its pharmaceutically acceptable or unacceptable salts or in a salt with a chiral acid which may be recrystallized to enhance its diastereoisomeric and or enantiomerc excess and provide the enantiomerically enriched cyclopropylamine (**Ia**) with a high trans/cis ratio or a salt thereof.

The cyclopropylamine (**Ia**), or one of its salts, may be further converted, in an additional step e'), into Ticagrelor, or in an intermediate useful for its synthesis or one of its salts.

A particularly preferred object of this invention is a process for preparing the *trans-*cyclopropylamine (**1**) wherein the stereocenters assume the configuration as depicted in the formula:

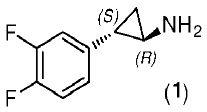

The β-nitroalcohols (**E**), (**Ea**), (**III**) or (**IIIa**) that are the reactants in steps b) or b'), may be converted, in the intermediate steps named path 2 and 3 (described in the following), into the 3-phenyl-substituted nitropropane compounds (**F**), (**Fa**), (**VI**) or (**VIa**): 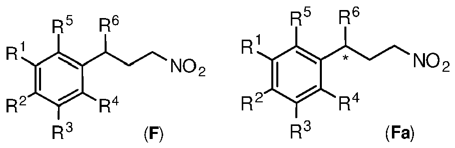 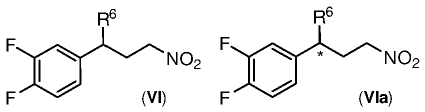 wherein R⁶ represents one of the leaving groups, known to the person skilled in the art, able to undergo an intramolecular nucleophilic substitution, such as for example, a mesylate, a tosylate, a halogen, a triflate, a nonaflate, a fluorosulfonate, or a nosylate.

A second aspect of the present invention are the new intermediates (**II**), (**III**), (**IV**), (**VI**) and the enantiomerically pure isomers thereof.

### Detailed description of the invention

All terms as used in this application, unless otherwise stated, shall be understood in their ordinary meaning as known in the art. Other more specific definitions for certain terms are used in the present application are set forth below and are intended to apply uniformly through-out the specification and claims unless an otherwise expressly set out definition provides a broader definition.

The compounds prepared by the processes of the present invention may have one or more stereogenic centers and may exist and may be used or isolated in optically active (enantiomeric or diastereoisomeric) and racemic forms. It is to be understood that the processes of the present invention can give rise to any racemic- or optically- active forms, or mixture thereof. It is to be further understood that the products of the invention processes can be isolated as racemic, or optically active forms, or mixtures thereof. Purification and characterization procedures for such products are known to those of ordinary skill in the art, and include recrystallization techniques, as well as chiral chromatographic separation procedures as well as other methods.

The sign "*" (asterisk) present in some formuale of this description indicate stereogenic (chiral) center, although the absence of asterisks does not necessarily imply that the compound lacks a stereocenter. Such formulae may refer to the racemate or to individual enantiomers or diastereoisomers, which may or may not be substantially pure.

A mixture of (*R*,*S*) enantiomers can contain the two single enantiomers in any ratio to each other. The enantiomeric purity is generally expressed as "enantiomeric excess" or e.e. and is defined, for example for the (*S*) enantiomer, as [(*S*-*R*)/(*R*+*S*)]x100, wherein *S* and *R* are respectively the amounts of the (*S*) and (*R*) enantiomers (as for example determined by chiral GLC or HPLC analysis or polarimetry).

The term "racemic" refers to a sample of a chiral compound which contains both the (+) and (-) isomers in equal amount.

The term "enantiomerically enriched" as used herein means that one of the enantiomers of a compound is present in excess compared to the other enantiomer. Moreover the term "high trans/cis ratio" as used herein means that one of the diastereoisomer (trans) of a compound is present in high excess compared to the other diasteroisomer (cis).

The term "single (*S*)- or (*R)*-enantiomer" means that the enantiomeric purity is usually at least about 96%, preferably at least 99%.

The compounds obtained by the chemical transformations of the present invention can be used for the following steps without further purification or can be effectively separated and purified by employing a conventional method well known to those skilled in the art, such as recrystallization, column chromatography, or by transforming them into a salt or by washing with an organic solvent or with an aqueous solution, optionally adjusting pH.

It will be understood that any compound described herein may also describe any pharmaceutically acceptable salts and or any pharmaceutically unacceptable salts thereof.

β-Nitroketone (**D**), the starting material for the synthetic route proposed in this application, can be prepared for example with the process depicted in the scheme below: 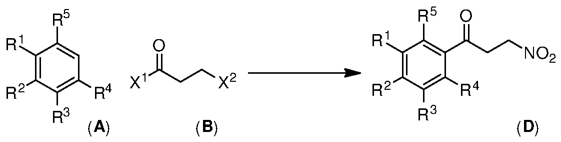 wherein the substituents assume the meanings given above and X¹ and X², independently from each other, are a halogen or a nitro group (with the condition that X¹ and X² cannot be simultaneously a nitro group).

When X¹ and X² are both a halogen, the β-nitroketone (**D**) can be prepared by Friedel-Crafts acylation followed by displacement of the halide with the nitrite anion (NO₂⁻).

In the first step (Friedel-Crafts acylation) the arene of general formula (**A**) is treated with a propionic acid derivative (**B**) (wherein X¹ and X², independently from each other are a halogen) in presence of a catalyst that may be a protic acid such as polyphosphoric acid or preferably H₂SO₄ or H₃PO₄, or a Lewis acid, such as, for example AlCl₃, AlBr₃, AlI₃, lanthanide triflates, zeolites, FeCl₃, and ZnCl₂; the catalyst is used in a quantity comprised between 1 and 1.5 equivalents, preferably 1.1 equivalents, compared to the molar quantity of the arene (**A**). The reaction can be performed in an apolar aprotic solvent, such as chlorinated solvents (preferably dichloromethane, dichloroethane, chlorobenzene or CCl₄ or a mixture thereof), at a temperature in the range comprised between room temperature and the reflux temperature of the solvent used, to yield the β-halopropriophenone (**C**): 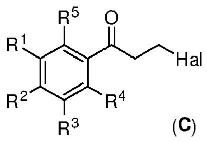 wherein the substituents have the meaning given above.

The quantity of the propionic acid derivative (**B**) used is comprised between 1 and 1.5 equivalents, preferably 1.1 equivalents, compared to the molar quantity of the arene (**A**).

Subsequently β-halopropriophenone (**C**) can be transformed into the β-nitroketone (**D**) with one of the methods known in the field, for example, by treating with a nitrite (preferably NaNO₂, KNO₂, Ca(NO₂)₂ or AgNO₂) in an aprotic polar solvent (preferably acetone, dimethylsulfoxide, dimethylformamide, dimethylacetamide, *N-*methylpyrrolidone or a mixture thereof).

Alternatively the reaction can be carried out with a nitrite salt under phase-transfer conditions using water immiscible solvents such as toluene or dichloromethane, water and a phase transfer catalyst, for example a quaternary ammonium salt.

The reaction can be performed at a temperature in the range comprised between room temperature and the reflux temperature of the solvent used. The quantity of nitrite used in the reaction can vary in a range comprised between 1 and 1.5 equivalents, preferably 1.25 equivalents, referred to the molar quantity of the β-halopropriophenone (**C**).

When in the propionic acid derivative (**B**) is X¹ = halogen and X² = NO₂, β-nitroketone (**D**) can be prepared treating the arene (**A**) with said propionic acid derivative (**B)** under Friedel-Crafts acylation conditions.

The synthetic route of the invention entails, as the first step, the reduction of the β-nitroketone (**D**) either in non-enantioselective reduction conditions, to provide a β-nitroalcohol (**E**) (synthetic path a1), or in enantioselective conditions, to provide an enantiomerically enriched β-nitroalcohol (**Ea**) (synthetic path a2).

The non-enantioselective reduction (a1) can be performed with one of the method known in the field, for example, by treating β-nitroketone (**D**) with a ketone reducing agent in a polar solvent, protic or aprotic, such as an alcohol (preferably methanol, ethanol) or an ether (preferably tetrahydrofuran), or a mixture thereof, optionally in mixture with water.

Suitable ketone reducing agents are those normally used in the field, see e.g. Burke-Danheiser, Handbook of Reagents for Organic Synthesis: Oxydizing and Reducing Agents, John Wiley & Sons (1999), for example, boron-containing reducing agents (preferably 9-BBN, borane, borane complex with dimethylsulfide, NaBH₄, LiBH₄, KBH₄, Super Hydride^{®}, NaCNBH₃ or Na(OAc)₃BH).

This reduction could be also performed using a metal alkoxide (preferably aluminium isopropoxide) in an alcohol (e.g. isopropanol), referred to as Meerwein-Ponndorf-Verley reaction or by treating under H₂ atmosphere with Pd/C, Pt/C, Pd/Al₂O₃ or Rh/Al₂O₃, provided that the suitable conditions are employed to avoid reduction of the nitro group.

Alternatively the β-nitroketone (**D**) can be enantioselectively reduced (a2) to an enatiomerically enriched β-nitroalcohol (**Ea**) with one of the methods known in the field, such as chemical methods or chemoenzymatic methods (as described for instance in Science of Synthesis: Stereoselective Synthesis 2, Chapter 2.3, Thieme (2011)).

Examples of the suitable chemical methods are, for example, the asymmetric Meerwein-Ponndorf-Verley reduction (as described in Cha, J. S., Org. Process. Res. Dev. (2006) 10, 1032) or the asymmetric transfer hydrogenation catalyzed by Group 8 or Group 9 metal complexes (such as for example using bifunctional ruthenium catalyst as described in Hashiguchi, S., et al., J. Am. Chem. Soc. (1995) 117, 7562). The enantioselective transfer hydrogenation can be also catalyzed by tethered ruthenium catalyst or diphosphinite ruthenium catalyst (such as those containing BINOL as chirality source). Another group of possible and convenient available ruthenium and rhodium catalysts for asymmetric transfer hydrogenation of ketones is based on pseudo-peptide ligands, derived from L-□-aminoacids.

A further example of asymmetric ketone reduction (known as Noyori asymmetric hydrogenation) entails the use of ruthenium-based catalysts in presence of a phosphine ligand, such as BINAP-RuX₂ catalysts or [(phosphine) RuX₂ (diamine)] complexes. Many phosphine ligands can be utilized in Noyori asymmetric ketone hydrogenation. Those normally used for the aim are biarylphosphines such as PhanePhos, QuinaPhos or BINAP derived phosphine (such as Xyl-BINAP or Tol-BINAP) or P-Phos derived ligand (for example P-Phos, Tol-P-Phos, Xyl-P-Phos). Phosphines which are not based on the BINAP model are especially effective ligands when used in combination with 1,2-diamine ligands such as DPEN (1,2-diphenylethylenediamine) and DAIPEN (1,1-bis(4-methoxyphenyl)-3-methyl-1,2-butanediamine).

A further enantioselective ketone reduction method, known as hydrosilylation of a carbonyl group, entails the addition of a Si-H bond to a carbonyl group, followed by hydrolysis of the initially formed silyl ether. Suitable catalysts for the asymmetric hydrosilylation of prochiral ketones are complexes of rhodium, ruthenium, iridium, copper, iron, tin, titanium or zinc.

The reduction of ketones with borane/chiral-oxazaborolidines is another widely used method for the asymmetric synthesis of secondary alcohols and is referred to as the Corey-Bakshi-Shibata reduction. Various immobilized diphenyl prolinol derivatives (oxazaborolidines), such as polymer- and silica-supported systems, dendrimer-based assemblies and fluorous compounds, have been developed as recyclable precatalysts. Borane-tetrahydrofuran, borane-dimethyl sulfide complex, cathecol borane, and borane-tetrahydrofuran solution stabilized by *N*-ethyl-*N*-isopropylamine are the most common borane reagents used in oxazaborolidine-catalyzed reduction of ketones. Preferred catalysts for the aim are the (*R*)-(+)-2-methyl-CBS-oxazaborolidine and (*S*)-(-)-2-methyl-CB S-oxazaborolidine.

Chirally modified boranes and aluminium hydrides are others widely used reagents to achieve high enantioselectivity in the reduction of prochiral ketones. Suitable boron-and aluminium-based chiral reducing agents are for example Alpine-borane^{®}, □-chlorodiisopinocampheylborane, NB-enantride, BINAL-H or NaBH₄/cobalt(II) complexes.

Examples of chemoenzymatic methods for the enantioselective transformation of prochiral carbonyl functional groups to the corresponding alcohols include the use of enzymes such as alcohol dehydrogenase (ADH), ketoreductase (KRED) or carbonyl reductase (CRED). Early approaches to accomplish bioreductions of carbonyl groups are based on the use of living wild-type microorganism.

Alcohol dehydrogenases utilize a stoichiometric cofactor that delivers the reduction equivalents. Either nicotinamide adenine dinucleotide (NADH) or nicotinamide adenine dinucleotide phosphate (NADPH) fulfils the function of hydride donor. The oxidized cofactor uncoupled from enzyme is reduced again *in situ* by a reductive cofactor regeneration system (which could be an enzyme-based or chemical-based reduction system). Examples of chemical-based reductive cofactor regeneration include achiral rhodium complexes with bipyridine and phenanthroline ligands compatible with alcohol dehydrogenases. Examples of enzyme-coupled cofactor recycling methods are based on the use, for example, of D-glucose and its oxidative transformation into D-gluconolactone catalyzed by a glucose dehydrogenase or on the use of a formate dehydrogenase necessary to oxidize formate to CO₂.

Suitable enzymes for the asymmetric reduction of ketones are for example YMR226c (alcohol dehydrogenase from *Saccharomyces cerevisiae*), ADH-RR-A (alcohol dehydrogenase-A from *Rodococcus ruber*), ADH-PFUR (alcohol dehydrogenase from *Pyrococcus furiosus*), PAR (phenylacetaldehyde reductase from *Corynebacterium*), ADH-RAS (alcohol dehydrogenase from *Ralstonia sp*.), ADH-PP (alcohol dehydrogenase from *Paracoccus pantotrophus*), ADH-PM (alcohol dehydrogenase from *Pichia methanolica*), Geo (dried cells of *Geotrichum candidum*), KRED 102, KRED 105, KRED 111, KRED 112, KRED 113, KRED 119, KRED 123, KRED 124, KRED 130, KRED 110, KRED 101, KRED 131, KRED 107, KRED 118, ADH-CM (*Candida magnoliae* alcohol dehydrogenase), ADH-LS (*Leifsonia* alcohol dehydrogenase), Geo-APG4 (dried cells of *Geotrichum candidum* (APG₄)) or ADH-HP (cloned alcohol dehydrogenase from *Hansenula polymorpha*) Geo (dried cells of *Geotrichum candidum*). A preferred ketoreductase for the aim of the invention is KRED 110 using NADH as cofactor.

Wild-type whole-cell reductions include the use of their metabolism for cofactor regeneration, therefore avoiding an external regeneration system. Alternatively isopropanol can be added as the source of hydride. Some of the whole-cell biocatalysts for the asymmetric reductions of carbonyl compounds to chiral alcohols are, for example, *Saccharomyces cerevisiae* (baker's yeast), *Geotrichum candidum*, *Candida magnoliae, Acenitobacter calcoaceticus, Candida maris, Rhodococcus sp., Streptomyces nodosus*, *Leuconostoc onenos*, *Nerospora crassa*, *Candida boidinii* and *Phichia methanolica, Zygosaccharomyces rouxii, Microbacterium camphoquemadoensis* and *Mucor hiemalis* or *Candida sorbophila.*

The next step of the synthetic route herein described is the cyclization of the β-nitroalcohols (**E**) or (**Ea**) to provide the corresponding nitrocyclopropanes (**G**) or (**Ga**) with a high trans/cis ratio following three possible synthetic paths, named path 1, path 2 and path 3 according to the scheme below:

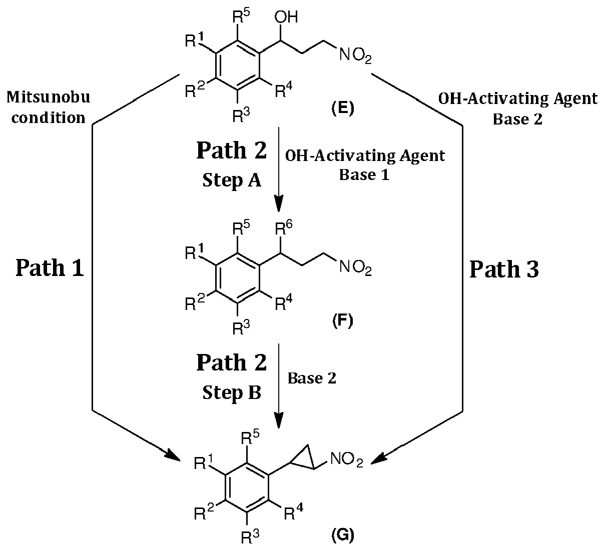

Base 1 and Base 2 are one of the bases known in the field as useful for the aim, as detailed below.

Path 1 entails the nitrocyclopropane formation by an intramolecular Mitsunobu reaction.

Such reaction conditions comprise reacting β-nitroalcohol (**E)** or (**Ea**) in presence of a phosphine (preferably triphenylphosphine or tri-*n*-butylphosphine) with an azodicarboxylate reagent (preferably diethyl azodicarboxylate or diisopropyl azodicarboxylate) in an apolar or in a polar solvent, preferably an aprotic polar solvent, such as dichloromethane or tetrahydrofuran or a mixture thereof; this reaction is carried out preferably at a temperature in the range comprised between 0 °C and 25 °C.

The quantity of the phosphine and of the azodicarboxylate used is comprised between 1 and 2 equivalents, preferably 1.5 equivalents, compared to the molar quantity of the β-nitroalcohol (**E**) or (**Ea**).

Path 2 step A entails the transformation of the β-nitroalcohol (**E**) or (**Ea**) in a 3-phenyl-substituted nitropropane (**F**) or (**Fa**) as depicted below: 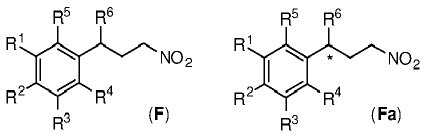 wherein the substituents can assume the meanings given above.

When in the 3-phenyl-substituted nitropropane (**F**) or (**Fa**) R⁶ is a sulphonate, such as mesylate, trifluoromethanesulfonate (triflate) or tosylate, its preparation can be performed with one of the methods generally known in the field, for example by treating with the corresponding sulfonyl halide or sulfonyl anhydride, in presence of an organic base (named Base 1), preferably a tertiary amine (cyclic or acyclic tertiary amine), such as triethylamine, *N*,*N*-diisopropylethylamine, *N,N-*diisopropylmethylamine, *N*-methylpyrrolidine, *N*-methylmorpholine or *N,N-*dicyclohexylmethylamine, *N*,*N*-diethylaniline. This reaction can also be performed using stronger amine bases (such as 1,8-Diazabicyclo[5.4.0]undec-7-ene (DBU) or 1,5-Diazabicyclo[4.3.0]non-5-ene (DBN)) or pyridines (such as pyridine, 2-methylpyridine, 2,6-dimethylpyridine, 2,4,6-trimethylpyridine or 4-dimethylaminopyridine), in an appropriate solvent, such as an apolar solvent or a protic polar solvent (particularly preferred solvents are dichloromethane, toluene, ethyl acetate, tetrahydrofuran or a mixture thereof).

The quantity of the sulfonyl halide or of the sulfonyl anhydride used is comprised between 1 and 1.5 equivalents, preferably 1.2 equivalents, compared to the molar quantity of the β-nitroalcohol (**E**) or (**Ea**). The amount of the organic base used is comprised between 1 and 3 equivalents, preferably 1.5 equivalents, compared to the molar quantity of the β-nitroalcohol (**E**) or (**Ea**).

Alternatively, when in the 3-phenyl-substituted nitropropane (**F**) or (**Fa**) R⁶ is a halogen, said compound can be prepared by treating the β-nitroalcohol (**E**) or (**Ea**) with a molecular halogen X₂ (preferably Cl₂, Br₂ or I₂) or with a halogenating agent such as CX₄ (preferably CBr₄, CCl₄), in presence of stechiometric amount of a phosphine (preferably triphenylphosphine) in a polar or apolar protic solvent, such as dichloromethane or tetrahydrofuran or a mixture thereof, at a temperature in the range comprised between -10 °C and 10 °C, preferably 0 °C.

The quantity of the molecular halogen or of the halogenating agent are comprised between 1 and 2 equivalents compared to the molar quantity of the β-nitroalcohol (**E**) or (**Ea**), preferably 1.5 equivalents. The quantity of the phosphine is comprised between 1 and 2 equivalents compared to the molar quantity of the β-nitroalcohol (**E**) or (**Ea**), preferably 1.5 equivalents.

Said 3-phenyl-substituted nitropropane (**F**) or (**Fa**) wherein R⁶ is a halogen can be also prepared by treating the β-nitroalcohol (**E**) or (**Ea**) with thionyl chloride or thionyl bromide in an apolar aprotic solvent, such as dichloromethane, toluene, acetates (preferably alkylacetates) or a mixture thereof, optionally in presence of an organic base (preferably pyridine).

Alternatively said compounds can be prepared from β-nitroalcohol (**E**) or (**Ea**) previously activated, for example in the form of a mesylate or a tosylate, by treating with an alkaline halide, such as for example sodium iodide or bromide, in an aprotic polar solvent (preferably dimethylformamide, dimethylacetamide, acetonitrile, methylethylketone or a mixture thereof), at a temperature in the range comprised between room temperature and the reflux temperature of the solvent used.

The quantity of the alkaline halide used is comprised between 1 and 5 equivalents, compared to the molar quantity of the 3-phenyl-substituted nitropropane (**F**) or (**Fa**). Path 2 step B entails a cyclization reaction to provide the nitrocyclopropane (**G**) or (**Ga**) starting from the 3-phenyl-substituted nitropropane (**F**) or (**Fa**).

This reaction can be carried out, for example, by treating 3-phenyl-substituted nitropropane (**F**) or (**Fa**), in a suitable solvent, such as an apolar or an aprotic polar solvent (preferably toluene, tetrahydrofuran, dichloromethane or a mixture thereof) under basic conditions at a temperature in the range comprised between -78 °C and the reflux temperature of the solvent used (preferably comprised between -20 °C and 30 °C).

Appropriate basic conditions are those that are capable of deprotonating the carbon directly connected to the nitro group (Cα). Such conditions can be obtained using one of the bases (Base 2) normally used in the field, which could be organic or inorganic bases (preferably DBU, DBN, 1,4-diazabicyclo[2.2.2]octane (DABCO), *N,N,N',N'-*tetramethylguanidine, or carbonates such as Na₂CO₃, K₂CO₃ or hydrides such as LiH or preferably NaH).

Path 3 involves the preparation of nitrocyclopropane (**G**) or (**Ga**) starting from β-nitroalcohol (**E**) or (**Ea**), via the formation of the 3-phenyl-substituted nitropropane (**F**) or (**Fa**) which are one-pot cyclized in presence of a base (namely Base 2) to yield the desired products.

This reaction can be performed using, as organic bases necessary for the preparation of the 3-phenyl-substituted nitropropane (**F**) or (**Fa**), the same bases which are able to promote the subsequent cyclization to provide nitrocyclopropane (**G**) or (**Ga**).

The following step of the synthetic pathway includes the reduction of the nitrocyclopropane (**G**) or (**Ga**) to the corresponding cyclopropylamine (**H**) or (**Ha**).

This reductive step can be performed treating nitrocyclopropane (**G**) or (**Ga**) in one of the conditions known to the skilled person, such as for example by treating the nitrocyclopropane (**G**) or (**Ga**) optionally solubilized in a polar solvent, protic or aprotic, such as alcohols (preferably methanol or 2-propanol) or ethers (preferably tetrahydrofuran) or a mixture thereof, with a metal (preferably Fe and Zn) in presence of acids, such as acetic acid or hydrochloric acid. Preferred conditions include the use of Zn and acetic acid or hydrochloric acid or Fe and acetic acid.

When cyclopropylamine (**H**) with a high trans/cis ratio is obtained as racemic mixture optionally the synthetic pathway of the invention includes a resolution step to provide the enantiomerically enriched cyclopropylamine (**Ha**) with a high trans/cis ratio or one of its salts.

The resolution can be performed with one of the methods known to person skilled in the art, e.g. according to the international patent application WO 00/34283, or via the formation of diastereoisomeric salts, by treatment with (+)-*O*-Acetyl-L-mandelic Acid, (-)-*O*-Acetyl-D-mandelic Acid, D-Aspartic Acid, L-Aspartic Acid, (+)-*cis*-2-Benzamidocyclohexanecarboxylic Acid, (-)-*cis*-2-Benzamidocyclohexanecarboxylic Acid, (*R*)-(-)-1,1'-binaphtyl-2,2'-diyl Hydrogenphosphate, (*S*)-(+)-1,1'-Binaphtyl-2,2'-diyl Hydrogenphosphate, (+)-Camphoric Acid, (+)-10-Camphorsulfonic Acid, (-)-10-Camphorsulfonic Acid, (-)-Diacetyl-L-tartaric Acid, (+)-Dibenzoyl-D-tartaric Acid, (-)-Dibenzoyl-L-tartaric Acid, (*R*)-(-)-*N*-(3,5-Dinitrobenzoyl)-a-phenylglycine, (+)-Di-*p*-toluoyl-D-tartaric Acid, (-)-Di-*p*-toluoyl-L-tartaric Acid, D-Glutamic Acid, L-Glutamic Acid, D-(+)-Malic Acid, L-(-)-Malic Acid, D-(-)-Mandelic Acid, L-(+)-Mandelic Acid, (*S*)-(+)-2-(6-Methoxy-2-naphthyl)propionic Acid, (*R*)-(+)-(α-Methylbenzyl)phthalamic Acid, D-Pyroglutamic Acid, L-Pyroglutamic Acid, D-(-)-Quinic Acid, L-(+)-Tartaric Acid or D-(-)-Tartaric Acid.

Optical resolution takes place with a molar ratio racemic amine:chiral acid comprised between 1:0.5 and 1:1, preferably 1:1 in a protic or aprotic polar solvent such as an alcohol, an ester (e.g. ethyl acetate), a ketone or a mixture thereof. The enantiomeric excess of the amine can be enhanced by recrystallizing the salt with the chiral acid. Optionally enantiomerically enriched cyclopropylamine (**Ha**) with a high trans/cis ratio can be converted in Ticagrelor or one of its pharmaceutically acceptable salts or can form acid addition salt, for example with suitable inorganic acids, such as hydrohalic acids (e.g., hydrochloric acid, hydrobromic acid), sulfuric acid with replacement of one or both protons, phosphoric acid with replacement of one or more protons, or with organic carboxylic, sulfonic or phosphonic acids, for example acetic acid, propionic acid, glycolic acid, succinic acid, maleic acid, fumaric acid, (*S*)- or (*R*)- malic acid, D- or L-tartaric acid, citric acid, benzoic acid, cinnamic acid, mandelic acid, salicylic acid, orotic acid, methanesulfonic acid, benzensulfonic acid, 4-toluensulfonic acid.

In one embodiment, for isolation and purification purposes it is also possible to use pharmaceutically unacceptable salts.

The invention will be further illustrated by means of the following examples. In the examples, the symbol 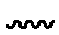 stands for a single bond of unspecified *R* or *S* configuration, and in general a formula containing said symbol indicates that the compound is obtained in racemic form.

### EXAMPLE 1

Preparation of 3-chloro-1-(3,4-difluorophenyl)propan-1-one (**4**), compound of formula (**C**) in which R¹=F, R²=F, R³=H, R⁴=H, R⁵=H, Hal=Cl.

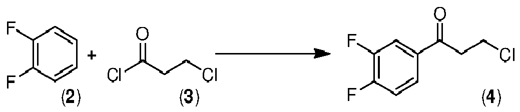

A mixture of 3-chloropropanoyl chloride (**3**) (12.2 g, 96.0 mmol) and 1,2-difluorobenzene (**2**) (10.0 g, 87.6 mmol) is added dropwise with stirring to a suspension of AlCl₃ (12.8 g, 96.0 mmol) in dichloromethane (30 mL), keeping the temperature below 30 °C with external cooling.

The mixture is stirred at room temperature for 24 hour (monitoring by TLC hexane/EtOAc 9:1).

After cooling to 0 °C, water is slowly added, separated from organic phase and extracted with dichloromethane. The collected organic phases are washed with a saturated solution of NaHCO₃ and subsequently dried over Na₂SO₄, filtered and evaporated under vacuum. The residue (brown oil) can be used in the next step as a crude or purified by flash chromatography, obtaining (**4**) (15.3 g, 85%) as a yellow oil.

¹H NMR (400 MHz, CDCl₃): δ 3.45 (t, *J* = 6.4 Hz, 2 H), 3.81 (t, *J* = 6.4 Hz, 2 H), 7.15-7.22 (m, 1H), 7.64-7.70 (m, 2 H) ppm.

¹³C NMR (100 MHz, CDCl₃): δ37.9, 40.5, 116.7 (d, *J* = 17.9 Hz), 117.1 (d, *J* = 17.6 Hz), 124.7, 133.1, 149.9 (dd, *J₁* = 249.6, *J₂* = 12.8 Hz), 153.3 (dd, *J₁* = 255.6, *J₂* = 12.7 Hz), 193.7 ppm.

### EXAMPLE 2

### Preparation of 1-(3,4-difluorophenyl)-3-nitropropan-1-one (5), compound of formula (D) in which R¹=F, R²=F, R³=H, R⁴=H, R⁵=H.

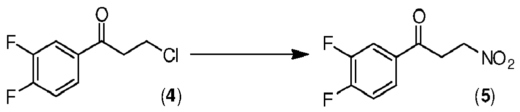

NaNO₂ (4.2 g, 60.8 mmol) is added to a stirred solution of the β-Chloropropiophenone (**4)** (10.0 g, 48.9 mmol) in acetone (400 mL) and maintained at 50 °C for 6 hours.

When the reaction is complete (monitoring by TLC hexane/EtOAc 7:3) the solvent is evaporated under vacuum and the residue is purified by flash chromatography, obtaining (**5**) (7.5 g, 71%) as a yellow solid.

¹H NMR (400 MHz, CDCl₃): δ 3.57 (t, *J* = 6.0 Hz, 2 H), 4.77 (t, *J* = 6.0 Hz, 2 H), 7.22-7.28 (m, 1 H), 7.72-7.75 (m, 2 H) ppm.

¹³C NMR (100 MHz, CDCl₃): δ 34.2, 68.6, 117.0 (d, *J* = 18.0 Hz), 117.4 (d, *J* = 17.9 Hz), 124.8, 132.3, 150.0 (dd, *J₁* = 249.6, *J₂* = 12.2 Hz), 153.6 (dd, *J₁* = 255.8, *J₂* = 12.4 Hz), 193.7 ppm.

### EXAMPLE 3

### Preparation of 1-(3,4-difluorophenyl)-3-nitropropan-1-ol (6), compound of formula (E) in which R¹=F, R²=F, R³=H, R⁴=H, R⁵=H.

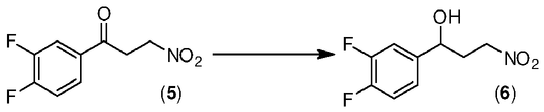

NaBH₄ (1.4 g, 37.0 mmol) is added to a 0 °C cooled solution of the β-nitroketone (**5**) (6.5 g, 30.2 mmol) in a mixture of THF (70 mL) and H₂O (70 mL). The mixture is stirred for 2 hours at room temperature, monitoring the reaction by TLC (hexane/EtOAc 7:3).

When the reaction is complete a saturated solution of NH₄Cl is added (until complete evolution of hydrogen), followed by an extraction with diethyl ether. The collected organic phases are dried over Na₂SO₄ filtered and evaporated under reduced pressure. The residue is purified by flash chromatography, obtaining (**6**) (6.0 g, 91%) as a colorless oil.

¹H NMR (400 MHz, CDCl₃): δ 2.19-2.38 (m, 2 H), 4.37-4.43 (m, 1H), 4.52-4.59 (m, 1H), 4.75 (dd, *J₁* = 8.8, *J₂* = 4.0 Hz, 1H), 7.01-7.17 (m, 3 H) ppm.

¹³C NMR (100 MHz, CDCl₃): δ 35.4, 69.5, 71.6, 114.2 (d, *J* = 17.5 Hz), 117.1 (d, *J* = 17.3 Hz), 121.2, 139.7, 148.5 (dd, *J₁* = 246.7, *J₂* = 12.6 Hz), 150.0 (dd, *J₁* = 246.9, *J₂* = 12.3 Hz) ppm.

### EXAMPLE 4

### Preparation of 4-(1-bromo-3-nitropropyl)-1,2-difluorobenzene (7), compound of formula (F) in which R¹=F, R²=F, R³=H, R⁴=H, R⁵=H, R⁶=Br.

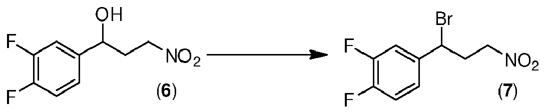

CBr₄ (27.5 g, 82.9 mmol) is added to a 0 °C solution of the β-nitroalcohol (**6**) (12.0 g, 55.3 mmol) and PPh₃ (21.7 g, 82.7 mmol) in THF (150 mL), maintaining the mixture at the same temperature for 1 hour.

When the reaction is complete (monitoring by TLC hexane/EtOAc 8:2), water is added, followed by an extraction with diethyl ether. The collected organic phases are dried over sodium sulphate, filtered and evaporated under vacuum. The residue is purified by flash chromatography, obtaining (7) (10.1 g, 65%) as a yellow oil.

¹H NMR (400 MHz, CDCl₃): δ 2.69-2.81 (m, 2 H), 4.46-4.62 (m, 2 H), 4.97 (dd, *J₁* = 9.2,

*J₂* = 6.0 Hz, 1H), 7.11-7.26 (m, 3 H) ppm.

¹³C NMR (100 MHz, CDCl₃): δ 36.4, 47.9, 72.8, 116.0 (d, *J* = 18.0 Hz), 117.4 (d, *J* = 17.1 Hz), 123.1, 137.0, 149.9 (dd, *J₁* = 246.9, *J₂* =12.4 Hz),150.1 (dd, *J₁* = 247.2, *J₂ =* 12.6 Hz) ppm.

### EXAMPLE 5

### Preparation of 1,2-difluoro-4-(2-nitrocyclopropyl)benzene (8), compound of formula (G) in which R¹=F, R²=F, R³=H, R⁴=H, R⁵=H.

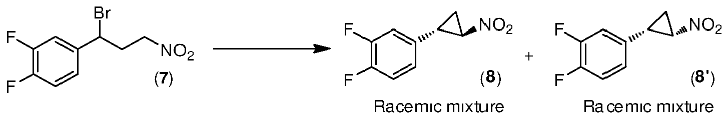

DBU (1.6 g, 10.5 mmol) is added to a -78 °C cooled solution of the 4-(1-bromo-3-nitropropyl)-1,2-difluorobenzene (7) (3.0 g, 10.7 mmol) in THF (50 mL), maintaining the mixture at the same temperature for 30 minutes (monitoring by TLC hexane/EtOAc 8:2). Subsequently HCl 1N is added until pH=2 and the reaction is warmed to room temperature. The phases are separated and the aqueous phase is extracted with diethyl ether. The collected organic phases are dried over Na₂SO₄, filtered and evaporated under vacuum.

The residue is analyzed by GC, showing a trans/cis (**8/8'**) ratio of 9:1 determined using the following GC method:

| | |
|---|---|
| **Gas Chromatograph** | Varian 3900 |
| **Column** | Capillary column VF-5ms, 30 m, 0.25 mm, 0.25 µm. |
| **Method** | 5 min at 80 °C, 20 min gradient from 80 °C to 280 °C, |
| | 20 min at 280 °C |
| **Retention time (8)** | 13.7 min |
| **Retention time (8')** | 14.0 min |

The crude reaction mixture is then purified by flash chromatography with gradient elution hexane/EtOAc 100:0 → 50:50, obtaining pure (**8**) (R_{f}=0.62 hexane/EtOAc 8:2) (1.6 g, 75%) as a yellow oil and pure (**8'**) (R_{f}=0.45 hexane/EtOAc 8:2) (50 mg) as a yellow oil.

Trans isomer (**8**) ¹H NMR (400 MHz, CDCl₃): δ 1.61 (q, *J* = 7.2 Hz, 1 H), 2.24 (ddd, *J* = 10.6, 6.4, 4.0 Hz, 1 H), 3.09 (ddd, *J* = 10.7, 7.9, 2.9 Hz, 1H), 4.33-4.36 (m, 1 H), 6.85-6.94 (m, 2 H), 7.05-7.24 (m, 1 H) ppm.

¹³C NMR(100 MHz, CDCl₃): δ 18.1, 27.7, 60.9, 115.4 (d, *J* = 17.8 Hz), 117.1 (d, *J* = 17.4 Hz), 122.6, 133.1, 149.3 (dd, *J₁* = 247.3, *J₂* = 12.6 Hz), 149.0 (dd, *J₁* = 247.0, *J₂* = 12.3 Hz) ppm.

Cis isomer (**8'**) ¹H NMR (300 MHz, CDCl₃) δ 1.67 (dt, *J* = 9.6, 6.8 Hz, 1H), 2.28 (ddd, *J* = 8.9, 7.0, 4.2 Hz, 1H), 2.78 (q, *J* = 8.9 Hz, 1H), 4.61 (ddd, *J* = 8.2, 6.7, 4.2 Hz, 1H), 6.94 -7.22 (m, 3H) ppm

### EXAMPLE 6

### Preparation of 2-(3,4-difluorophenyl)cyclopropanamine (1'), compound of formula (H) in which R¹=F, R²=F, R³=H, R⁴=H, R⁵=H.

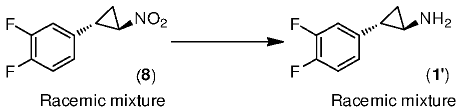

Zinc dust (3.2 g, 48.9 mmol) is added to a solution of the nitrocyclopropane (**8**) (1.0 g,

5.0 mmol) in acetic acid (50 mL). The mixture is stirred at room temperature for 24 hours (monitoring by TLC CHCl₃/MeOH 9:1).

When the reaction is complete, it is filtered on a celite pad and washed with dicloromethane and water. The phases are separated and the aqueous phase is extracted with CH₂Cl₂.

The collected organic phases are washed with a saturated solution of NaHCO₃, dried over Na₂S0₄, filtered and evaporated under vacuum. The residue is purified by flash chromatography, obtaining (**1'**) (0.4 g, 47%) as a yellow oil.

¹H NMR (400 MHz, CDCl₃): δ 0.89 (q, *J* = 7.2 Hz, 1 H), 1.01-1.04 (m, 1 H), 1.77-1.82 (m, 1 H), 1.92 (bs, 2 H), 2.27-2.48 (m, 1 H), 6.69-6.77 (m, 2 H), 6.95-6.99 (m, 1 H) ppm.

¹³C NMR (100 MHz, CDCl₃): δ 18.0, 29.2, 34.9, 113.9 (d, *J* = 17.1 Hz), 116.4 (d, *J* = 17.0 Hz), 121.2, 139.1, 148.0 (dd, *J₁* = 243.5, *J₂* = 12.7 Hz), 149.8 (dd, *J₁* = 245.7, *J₂* = 12.3 Hz) ppm.

### EXAMPLE 7

### Preparation of (R)-1-(3,4-difluorophenyl)-3-nitropropan-1-ol (6a), compound of formula (Ea) in which R¹=F, R²=F, R³=H, R⁴=H, R⁵=H.

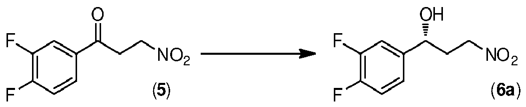

(*S*)-(-)-2-methyl-CBS-oxaborolidine (2.9 g, 10.5 mmol) is added to a 0 °C cooled solution of the β-nitroketone (**5**) (10.0 g, 46.5 mmol) in THF (70 mL). The mixture is cooled to -20 °C and a solution 2.0 M of borane-dimethylsulfide complex in THF (23 mL, 46.4 mmol) is added, maintaining the same temperature under stirring for 12 hours.

When the reaction is complete (monitoring by TLC hexane/EtOAc 7:3), the mixture is warmed to 0 °C and MeOH is added until complete gas evolution, followed by the addition of HCl 1N and EtOAc. The phases are separated and the aqueous phase is extracted with ethyl acetate.

The collected organic phases are dried over Na₂SO₄, filtered and evaporated under vacuum.

The residue is purified by flash chromatography, obtaining (**6a**) (7.6 g, 75%) as a colorless oil with an enantiomeric ratio of 97:3, monitored using the following HPLC method:

| | |
|---|---|
| **Column** | Chiralpak IB 250x4,6mm |
| **Flow rate** | 1 mL/min |
| **Injection volume** | 10 µL |
| **Wavelength** | 220 nm |
| **Column temperature** | RT |
| **Mobile phase: isocratic** | 99% Hexane, 1% Isopropanol |
| **Diluting solution** | Hexane /Isopropanol (9:1) (conc: 1 mg/mL) |
| **Retention time** | 61.3 min |

### EXAMPLE 8

### Preparation of 1,2-difluoro-4-((1S,2R)-2-nitrocyclopropyl)benzene (8a), compound of formula (Ga) in which R¹=F, R²=F, R³=H, R⁴=H, R⁵=H.

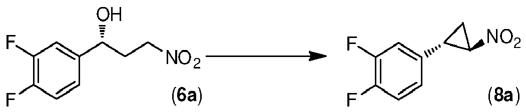

A solution of the β-alcohol (**6a**) (1.0 g, 4.6 mmol) in THF (50 mL) is treated at 0 °C with mesyl chloride (1.0 g, 9.2 mmol) followed by DBU (3.5 g, 23.0 mmol).

The mixture is stirred at the same temperature for 1 hour (trans/cis ratio 1:1, measured by GC method reported in example 5), then for 12 hours at room temperature (trans/cis 99:1, measured by GC method reported in example 5).

When the reaction is complete (monitoring by TLC hexane/EtOAc 8:2), HCl 1N and diethyl ether are added. The phases are separated and the aqueous phase is extracted with diethyl ether.

The collected organic phases are dried over Na₂SO₄, filtered and evaporated under vacuum.

The residue is purified by flash chromatography, obtaining pure (**8a**) (0.7 g, 76%) as a pale yellow oil with an enantiomeric ratio of 94:6 measured using the following HPLC method:

| | |
|---|---|
| **Column** | Chiralpak AD 250x4,6mm |
| **Flow rate** | 0.5 mL/min |
| **Injection volume** | 10 µL |
| **Wavelength** | 210 nm |
| **Column temperature** | RT |
| **Mobile phase: isocratic** | 95% Heptane, 5% Ethanol, 0.1% TFA |
| **Diluting solution** | Heptane /Ethanol (9:1) (conc: 1 mg/mL) |
| **Retention time** | 22.3 min |

### EXAMPLE 9

### Preparation of 1,2-difluoro-4-((1S,2R)-2-nitrocyclopropyl)benzene (8), compound of formula (Ga) in which R¹=F, R²=F, R³=H, R⁴=H, R⁵=H.

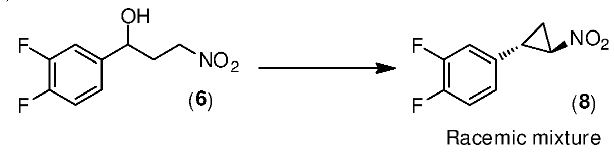

Triethylamine (1.8 mL, 12.9 mmol) is added dropwise at -15 °C to a solution of the β-nitroalcohol (**6**) (1.85 g, 8.5 mmol) and mesyl chloride (790 µL, 10.2 mmol) in THF (28 mL). The reaction is stirred at the same temperature for 2 hours monitoring by TLC (1:3 AcOEt-hexane). The mixture is filtered on a Celite pad to remove triethylamine hydrochloride. The filtrate is cooled to 0 °C and DBU (3.8 mL, 25.4 mmol) is added via a syringe pump in 1.5 hours. After 1 hour the reaction mixture is warmed to room temperature and stirred for 18 hours to achieve the isomerization to the trans isomer.

4N Hydrochloric acid is added until pH = 3, THF is evaporated under reduced pressure and the product is extracted with methyl *tert*-butyl ether to afford the product (containing 15% of starting material **6**) as a yellow oil with a trans/cis ratio of 98:2 using the GC method as reported in example 5.

### EXAMPLE 10

### Preparation of 1,2-difluoro-4-((1S,2R)-2-nitrocyclopropyl)benzene (8), compound of formula (Ga) in which R¹=F, R²=F, R³=H, R⁴=H, R⁵=H.

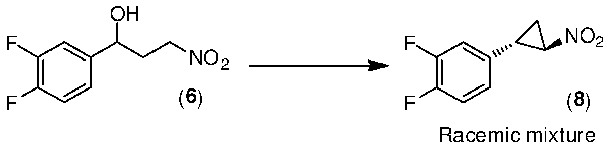

Triethylamine (1.8 mL, 12.9 mmol) is added dropwise at -15 °C to a solution of the β-nitroalcohol (1.85 g, 8.5 mmol) and mesyl chloride (790 µL, 10.2 mmol) in toluene (25 mL). The reaction is stirred at the same temperature for 2 hours monitoring by TLC (1:3 AcOEt-hexane). ¹H NMR of the crude reaction mixture shows complete conversion into the mesylate.

The mixture is washed with water and brine, the solvent is evaporated under reduced pressure and the residue is diluted with THF (25 mL). The solution is cooled to 0 °C and DBU (3.8 mL, 25.4 mmol) is added keeping the temperature below +5 °C. At the end of the addition the mixture is stirred at room temperature for 18 hours. 4N Hydrochloric acid is added until pH = 3, THF is evaporated under reduced pressure and the product is extracted with methyl *tert*-butyl ether to afford the product (1.52 g, 89% yield) as a yellow oil with a trans/cis ratio of 98:2 using the GC method as reported in example 5.

### EXAMPLE 11

### Preparation of (S)-1-(3,4-difluorophenyl)-3-nitropropan-1-ol (6b), compound of formula (Ea) in which R¹=F, R²=F, R³=H, R⁴=H, R⁵=H.

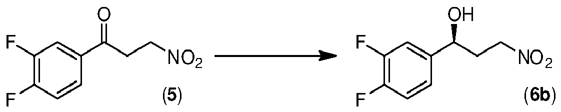

(*R*)-(+)-2-methyl-CBS-oxaborolidine (2.9 g, 10.5 mmol) is added to a 0 °C cooled solution of the β-nitroketone (**5**) (10.0 g, 46.5 mmol) in THF (70 mL). The mixture is cooled to -20 °C and a solution 2.0 M of borane-dimethylsulfide complex in THF (23 mL, 46.0 mmol) is added, maintaining the same temperature under stirring for 12 hours.

When the reaction is complete (monitoring by TLC hexane/EtOAc 7:3), the mixture is warmed to 0 °C and MeOH is added until complete gas evolution, followed by the addition of HCl 1N and EtOAc. The phases are separated and the aqueous phase is extracted with ethyl acetate.

The collected organic phases are dried over Na₂SO₄, filtered and evaporated under vacuum.

The residue is purified by flash chromatography, obtaining (**6b**) (7.6 g, 75%) as a yellow oil with an enantiomeric ratio of 97:3, measured using the following HPLC method:

| | |
|---|---|
| **Column** | Chiralpak IB 250x4,6mm |
| **Flow rate** | 1 mL/min |
| **Injection volume** | 10 µL |
| **Wavelength** | 220 nm |
| **Column temperature** | RT |
| **Mobile phase: isocratic** | 99% Hexane, 1% Isopropanol |
| **Diluting solution** | Hexane/Isopropanol (9:1) (conc: 1 mg/mL) |
| **Retention time** | 55.8 min |

### EXAMPLE 12

### Preparation of 4-(1-bromo-3-nitropropyl)-1,2-difluorobenzene (7), compound of formula (F) in which R¹=F, R²=F, R³=H, R⁴=H, R⁵=H, R⁶=Br.

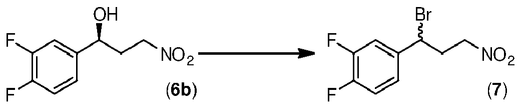

CBr₄ (27.5 g, 82.9 mmol) is added to a 0 °C solution of the β-nitroalcohol (**6b**) (12.0 g, 55.3 mmol) and PPh₃ (21.7 g, 82.7 mmol) in THF (150 mL), maintaining the mixture at the same temperature for 1 hour.

When the reaction is complete (monitoring by TLC hexane/EtOAc 8:2), water is added, followed by an extraction with diethyl ether. The collected organic phases are dried over sodium sulphate, filtered and evaporated under vacuum.

The residue is purified by flash chromatography, obtaining (7) (10.1 g, 65%) (yellow oil) as racemic mixture determined using the following HPLC method:

| | |
|---|---|
| **Column** | Chirapack AD column: 250 X 4.6 |
| **Flow rate** | 0.5 mL/min |
| **Injection volume** | 10 µL |
| **Wavelength** | 210 nm |
| **Column temperature** | RT |
| **Mobile phase: isocratic** | 96% Hexane, 4% isopropanol, 0.2% triethylamine, |
| | 0.2% trifluoroacetic acid |
| **Retention time** | 27.7 and 28.9 min |

### EXAMPLE 13

### Preparation of 1,2-difluoro-4-((1S,2R)-2-nitrocyclopropyl)benzene (8a), compound of formula (Ga) in which R¹=F, R²=F, R³=H, R⁴=H, R⁵=H.

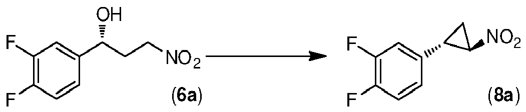

Mesyl chloride (1.0 g, 8.7 mmol) and N-methylmorpholine (2.3 g, 22.7 mmol) are added dropwise to a 0°C cooled solution of the β-nitroalcohol (**6a)** (1.0 g, 4.6 mmol) in THF (50 mL). The mixture is stirred at the same temperature for 1 hour (trans/cis ratio 1:1, measured by GC method reported in example 5), then for 12 hours at room temperature (trans/cis 1:1, measured by GC method reported in example 5).

When the reaction is complete (monitoring by TLC hexane/EtOAc 8:2), HCl 1N and diethyl ether are added. The phases are separated and the aqueous phase is extracted with diethyl ether.

The collected organic phases are dried over Na₂SO₄, filtered and evaporated under vacuum.

The residue is purified by flash chromatography, obtaining pure (**8a**) (0.3 g, 33%) as a pale yellow oil.

### EXAMPLE 14

### Preparation of 1,2-difluoro-4-((1S,2R)-2-nitrocyclopropyl)benzene (8a), compound of formula (Ga) in which R¹=F, R²=F, R³=H, R⁴=H, R⁵=H.

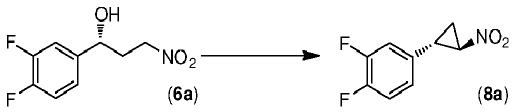

Mesyl chloride (1.0 g, 8.7 mmol) and DBU (2.3 g, 15.1 mmol) are added dropwise to a 0°C cooled solution of the β-alcohol (**6a**) (1.0 g, 4.6 mmol) in DMF (50 mL). The mixture is stirred at the same temperature for 1 hour (trans/cis ratio 1:1, measured by GC method reported in example 5), then for 12 hours at room temperature (trans/cis 99:1, measured by GC method reported in example 5).

When the reaction is complete (monitoring by TLC hexane/EtOAc 8:2), HCl 1N and diethyl ether are added. The phases are separated and the aqueous phase is extracted with diethyl ether.

The collected organic phases are dried over Na₂SO₄, filtered and evaporated under vacuum.

The residue is purified by flash chromatography, obtaining pure (**8a**) (0.5 g, 54%) as a pale yellow oil with an enantiomeric ratio of 96:4 monitored using the HPLC method described in example 8.

### EXAMPLE 15

### Preparation of (R)-1-(3,4-difluorophenyl)-3-nitropropan-1-ol (6a), compound of formula (Ea) in which R¹=F, R²=F, R³=H, R⁴=H, R⁵=H_{.}

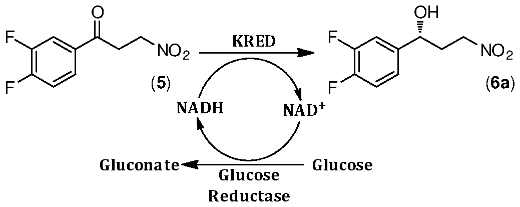

In a 2 mL Eppendorf tube, β-nitroketone (5) (2.5 mg, 0.01 mmol) in DMSO (50 µL) was dissolved in phosphate buffer (0.5 mL, 0.1 M, pH = 7.0). The solution was added of 0.5 mg of NADH, 0.5 mg of Glucose Reductase, 2.5 mg of KRED 110 and 2.6 mg Glucose. The tube was placed in a shaking incubator and shaken at 30 °C and 250 rpm. The reaction was monitored by HPLC (according to the method reported in example 8) at 6, 24, 48 and 86 hours with a final conversion of 63% by ¹H NMR and e.e. of 80% by chiral HPLC.

## Claims

1. A process for preparing a nitrogen substituted cyclopropane, comprising the following steps:
a) reducing a β-nitroketone (**D**) 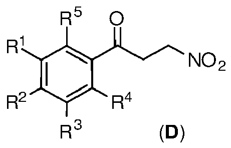 in which the substituents R¹, R², R³, R⁴, R⁵ are, independently from each other, hydrogen or a halogen, according to either:
a1) non-enantioselective reduction conditions, to provide a β-nitroalcohol (**E**): 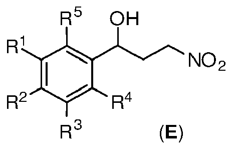 or:
a2) enantioselective reduction conditions, to provide an enantiomerically enriched β-nitroalcohol (**Ea**) in which (*) denotes a stereocenter: 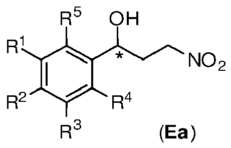
b) cyclizing the β-nitroalcohol (**E**) to yield a nitrocyclopropane (**G**) with a high trans/cis ratio, or the enantiomerically enriched β-nitroalcohol (**Ea**) to yield an enantiomerically enriched nitrocyclopropane (**Ga**), in which (*) denotes a stereocenter, with a high trans/cis ratio: 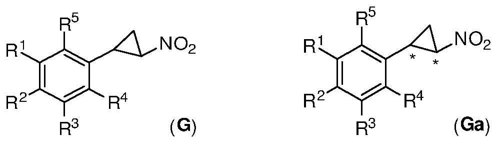
c) reducing the nitrocyclopropane (**G**) to the corresponding cyclopropylamine (**H**) with a high trans/cis ratio or a salt thereof, or the enantiomerically enriched nitrocyclopropane (**Ga**) to the enantiomerically enriched cyclopropylamine (**Ha**), in which (*) denotes a stereocenter, with a high trans/cis ratio, or a salt thereof:

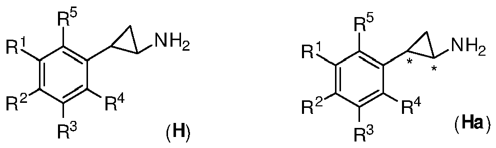

2. Process according to claim 1 in which, when said step a) is carried out according to non-enantioselective reduction conditions a1), an additional step d) is performed, to convert the cyclopropylamine (**H**) into a salt with a chiral acid, said salt capable to undergo recrystallization to enhance its diastereoisomeric and/or enantiomeric excess and provide the enantiomerically enriched cyclopropylamine (**Ha**) with a high trans/cis ratio or a salt thereof.

3. Process according to any one of claims 1 or 2, in which the cyclopropylamine (**Ha**), or one of its salts, is further converted, in an additional step e), into Ticagrelor or in an intermediate useful for its synthesis or one of its salts.

4. Process according to any one of the preceding claims, in which substituents R¹ and R² are fluorine, substituents R³, R⁴ and R⁵ are hydrogen, the β-nitroketone subjected to reduction in step a) is the compound of formula (**IV**): 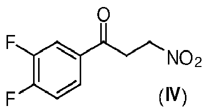 and the product resulting from the process is the cyclopropylamine of formula (**Ia**) with a high trans/cis ratio or a salt thereof: 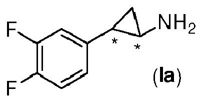 wherein (*) denotes a stereocenter.

5. Process according to any one of the preceding claims for preparing *trans-*cyclopropylamine (**1**), wherein the stereocenters assume the configuration as depicted in the following formula:

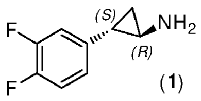

6. Process according to any one of the preceding claims, wherein step a) is carried out according to non-enantioselective reduction conditions a1), by treating β-nitroketone (**D**) either in a protic or aprotic polar solvent with a ketone reducing agent, or using a metal alkoxide in an alcohol or under a H₂ atmosphere with Pd/C, Pt/C, Pd/Al₂O₃ or Rh/Al₂O₃ as hydrogenation catalyst, provided that the reaction conditions are such to avoid reduction of the nitro group.

7. Process according to claim 6, wherein said reducing agent is selected among 9-BBN, borane, borane complex with dimethylsulfide, NaBH₄, LiBH₄, KBH₄, Super Hydride^{®}, NaCNBH₃ or Na(OAc)₃BH.

8. Process according to any one of claims 1 and 3-5, wherein step a) is carried out according to enantioselective reduction conditions a2) with a chemical method or a chemoenzymatic method.

9. Process according to claim 8, wherein said chemical method is selected among the asymmetric Meerwein-Ponndorf-Verley reduction; the Noyori asymmetric hydrogenation; the asymmetric transfer hydrogenation catalyzed by Group 8 or Group 9 metal complexes; the addition of a Si-H bond to a carbonyl group with a catalyst selected among complexes of rhodium, ruthenium, iridium, copper, iron, tin, titanium or zinc, followed by hydrolysis of the initially formed silyl ether; the enantioselective reduction of ketones with borane/chiral oxazaborolidines; and the reduction with chirally modified boranes and aluminium hydrides.

10. Process according to claim 9, wherein said chiral oxazaborolidines are chosen between (*R)*-(+)-2-methyl-CBS-oxazaborolidine and (*S*)-(-)-2-methyl-CBS-oxazaborolidine.

11. Process according to claim 8, wherein said chemoenzimatic method comprises the use of the enzymes alcohol dehydrogenase, ketoreductase or carbonyl reductase.

12. Process according to claim 11, wherein said ketoreductase is KRED 110 used along with NADH as cofactor

13. Process according to any one of the preceding claims, wherein the cyclization step b) takes place according to one of the three following paths:
- path 1) cyclopropane formation by an intramolecular Mitsunobu reaction, comprising reacting β-nitroalcohol (**E**) or (**Ea**) in presence of a phosphine with an azodicarboxylate reagent in an apolar or in a polar solvent;
- path 2) transformation of the β-nitroalcohol (**E**) into a 3-phenyl-substituted nitropropane (**F**), or of the enantiomerically enriched β-nitroalcohol (**Ea**) into an enantiomerically enriched 3-phenyl-substituted nitropropane (**Fa**): 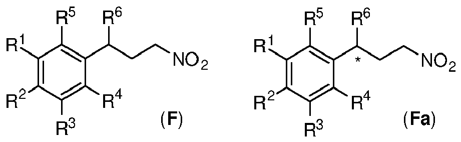 wherein R⁶ is a leaving group capable to undergo an intramolecular nucleophilic substitution, and (*) denotes a stereocenter, followed by a cyclization reaction carried out by treating 3-phenyl-substituted nitropropane (**F**) or (**Fa**), in an apolar or polar solvent with an organic or inorganic base (Base 2) selected among DBU, DBN, 1,4-diazabicyclo[2.2.2]octane (DABCO), *N*,*N*,*N'*,*N'*-tetramethylguanidine, or alkali metal carbonates;
- path 3) one-pot transformation of β-nitroalcohol (**E**) into a nitrocyclopropane of formula (**G**) via the formation of the 3-phenyl-substituted nitropropane (**F**), or one-pot transformation of enantiomerically enriched β-nitroalcohol (**Ea**) into an enantiomerically enriched nitrocyclopropane of formula (**Ga**) via the formation of the enantiomerically enriched 3-phenyl-substituted nitropropane (**Fa**), in which the base used for the formation of the 3-phenyl-substituted nitropropane (**F**) or (**Fa**) is the same base used in the cyclization reaction.

14. Process according to claim 13, wherein said leaving group R⁶ in path 2) is chosen among a halogen, a nosylate, a nonaflate, a triflate, a fluorosulfonate, a tosylate or a mesylate.

15. Process according to any one of the preceding claims, in which step c) takes place by treating either said nitro nitrocyclopropane (**G**) or said enantiomerically enriched nitrocyclopropane (**Ga**) with a metal in the presence of an acid.

16. Process according to claim 15, wherein said nitrocyclopropane (**G**) or enantiomerically enriched nitrocyclopropane (**Ga**) is solubilized in a protic or aprotic polar solvent, said metal is chosen between Fe and Zn, and said acid is chosen between acetic acid or hydrochloric acid.

17. Process according to any one of the preceding claims, in which the starting β-nitroketone (**D**) is prepared with the following reaction: 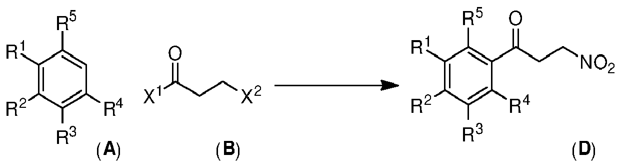 in which:
- the substituents R¹, R², R³, R⁴, R⁵ have the meanings given above;
- X¹ and X² are both a halogen,
and the β-nitroketone (**D**) is prepared by Friedel-Crafts acylation followed by displacement of the halide with the nitrite anion (NO₂⁻).

18. 1,2-difluoro-4-(2-nitrocyclopropyl)benzene and the enantiomerically pure isomers thereof.

19. 1-(3,4-difluorophenyl)-3-nitropropan-1-ol and the enantiomerically pure isomers thereof.

20. 1-(3,4-difluorophenyl)-3-nitropropan-1-one.

21. 3-phenyl-substituted nitropropane (**VI**) and the enantiomerically pure isomers thereof 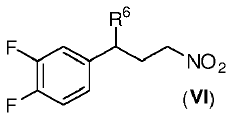 wherein R⁶ is chosen among a mesylate, a tosylate, a halogen, a triflate, a nonaflate, a fluorosulfonate, or a nosylate.
